(19) Europäisches Patentamt
European Patent Office
Office européen des brevets

(11) Veröffentlichungsnummer: **0 002 208**
**B1**

(12) **EUROPÄISCHE PATENTSCHRIFT**

(45) Veröffentlichungstag der Patentschrift: **11.03.81**

(21) Anmeldenummer: **78101379.2**

(22) Anmeldetag: **16.11.78**

(51) Int. Cl.³: **C 07 D 211/84**, C 07 D 211/90, C 07 D 401/04, C 07 D 401/12, A 61 K 31/44

(54) **Nitrosubstituierte 1,4-Dihydropyridine, diese enthaltende Arzneimittel sowie deren Herstellung.**

(30) Priorität: **26.11.77 DE 2752820**

(43) Veröffentlichungstag der Anmeldung: **13.06.79 Patentblatt 79/12**

(45) Bekanntmachung des Hinweises auf die Patenterteilung: **11.03.81 Patentblatt 81/10**

(84) Benannte Vertragsstaaten: **BE CH DE FR GB NL**

(56) Entgegenhaltungen: **Keine**

(73) Patentinhaber: **BAYER AG, Zentralbereich Patente, Marken und Lizenzen, D-5090 Leverkusen 1, Bayerwerk (DE)**

(72) Erfinder: **Bossert, Friedrich, Dr., Claudiusweg 7, D-5600 Wuppertal 1 (DE)**
Erfinder: **Franckowiak, Gerhard, Dr., Pahlkestrasse 17, D-5600 Wuppertal 1 (DE)**
Erfinder: **Heise, Arend, Dr., Moebeck 50, D-5600 Wuppertal 1 (DE)**
Erfinder: **Kazda, Stanislav, Dr., Pahlkestrasse 55N, D-5600 Wuppertal 1 (DE)**
Erfinder: **Meyer, Horst, Dr., Theodor-Heuss-Strasse 110, D-5600 Wuppertal 1 (DE)**
Erfinder: **Stoepel, Kurt, Dr., In den Birken 69, D-5600 Wuppertal 1 (DE)**
Erfinder: **Towart, Robertson, Dr., Claudiusweg 9, D-5600 Wuppertal 1 (DE)**
Erfinder: **Wehinger, Egbert, Dr., Donnenbergerstrasse 90, D-5620 Velbert 15 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchgebühr entrichtet worden ist (Art. 99(1) Europäisches Patentübereinkommen).



ACTORUM AG.

# Nitrosubstituierte 1,4-Dihydropyridine, diese enthaltende Arzneimittel sowie deren Herstellung

Die Erfindung betrifft neue nitrosubstituierte 1,4-Dihydropyridine, diese enthaltende Arzneimittel, insbesondere kreislaufbeeinflussende Mittel, sowie Verfahren zu ihrer Herstellung.

Es ist bereits bekannt geworden, dass man 2,6-Dimethyl-4-phenyl-1,4-dihydropyridin-3,5-dicarbonsäurediäthylester erhält, wenn man Benzylidenacetessigsäureäthylester mit β-Aminocrotonsäureäthylester oder Acetessigsäureäthylester und Ammoniak umsetzt (Knoevenagel, Ber. dtsch. chem. Ges. 31, 743 [1898]). Weiterhin ist bekannt, dass bestimmte 1,4-Dihydropyridine interessante pharmakologische Eigenschaften aufweisen (F. Bossert, W. Vater, Die Naturwissenschaften 58, 578 [1971]).

Weiterhin ist bekannt geworden, dass 1,4-Dihydropyridine, die in 3- und in 5-Position eine Estergruppierung tragen, pharmakologische Wirkungen zeigen (B. Loev et al. J. Med. Chem. 17, 956–965 [1974]).

Die Erfindung betrifft neue nitrosubstituierte 1,4-Dihydropyridine der allgemeinen Formel

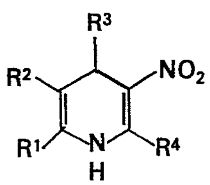

(I)

in welcher

$R^1$ und $R^4$ für einen Alkylrest mit 1 oder 2 Kohlenstoffatomen,

$R^2$ für eine Nitrogruppe oder für die Gruppe $COOR^6$ steht, wobei $R^6$ ein geradkettiges, verzweigtes, oder cyclisches Alkyl, Alkenyl oder Alkinyl mit bis zu 6 Kohlenstoffatomen bedeutet, und die Alkylgruppe gegebenenfalls durch ein Sauerstoffatom unterbrochen ist oder wobei die Alkylgruppe gegebenenfalls durch Halogen substituiert ist, und

$R^3$ für einen Phenylrest, der gegebenenfalls durch 1 oder 2 gleiche oder verschiedene Halogenatome, Nitro-, Cyano- oder Trifluormethylgruppen substituiert ist, oder für einen Pyridylrest stehen.

Die erfindungsgemässen Verbindungen weisen starke Coronarwirkung und antihypertensive Eigenschaften auf.

Weiterhin wurde gefunden, dass man die neuen nitrosubstituierten 1,4-Dihydropyridine der allgemeinen Formel I erhält, wenn man

A) Aldehyde der Formel

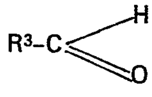

(II)

in welcher $R^3$ die oben angegebene Bedeutung hat mit

Enaminen der Formel

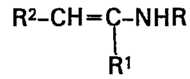

(III)

in welcher R, $R^1$ und $R^2$ die oben angegebene Bedeutung haben

und

Nitromethylketonen der Formel

$$R^4\text{-}CO\text{-}CH_2\text{-}NO_2 \qquad (IV)$$

in welcher $R^4$ die oben angegebene Bedeutung hat in Wasser oder in inerten organischen Lösungsmitteln umsetzt,

oder

B) Aldehyde der Formel

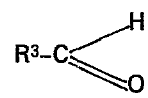

(II)

in welcher $R^3$ die oben angegebene Bedeutung hat mit

Carbonylverbindungen der Formel

$$R^1\text{-}CO\text{-}CH_2\text{-}R^2 \qquad (V)$$

in welcher $R^1$ und $R^2$ die oben angegebene Bedeutung haben

und

Nitroenaminen der Formel

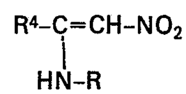

(VI)

in welcher R und $R^4$ die oben angegebene Bedeutung besitzen

in

Wasser oder inerten organischen Lösungsmitteln umsetzt

oder

C) Enamine der Formel

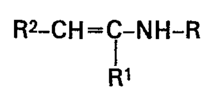

(III)

in welcher R, $R^1$ und $R^2$ die oben angegebene Bedeutung haben

mit

Nitroylidenverbindungen der Formel

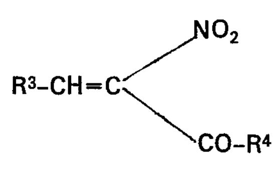

(VII)

in welcher $R^3$ und $R^4$ die oben angegebene Bedeutung haben
in Wasser oder inerten organischen Lösungsmitteln umsetzt
oder
D) Nitroenamine der Formel

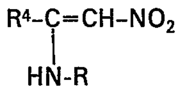
(VI)

in welcher R und $R^4$ die oben angegebene Bedeutung haben
mit
Ylidenketonen der Formel

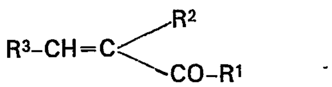
(VIII)

in welcher $R^1$, $R^2$ und $R^3$ die oben angegebene Bedeutung haben
in Wasser oder inerten organischen Lösungsmitteln umsetzt.

Die neuen erfindungsgemässen 1,4-Dihydropyridin-Derivate besitzen wertvolle pharmakologische Eigenschaften. Aufgrund ihrer kreislaufbeeinflussenden Wirkung können sie als Antihypertonika, als Vasodilatatoren sowie als Coronartherapeutika Verwendung finden.

Bei Kenntnis des Standes der Technik konnte nicht erwartet werden, dass der Austausch mindestens einer Estergruppierung in 3- oder 5-Position durch eine Nitrogruppe zu neuen Verbindungen führt, die vorteilhafte pharmakologische Eigenschaften besitzen. Es ist daher ausgesprochen überraschend, dass die erfindungsgemässen Verbindungen nach intravenöser Applikation am Hund bereits mit Dosierungen ab 0,03 mg/kg einen signifikanten Anstieg der Sauerstoffsättigung im Coronarsinus bewirken. Aufgrund dieser überraschend vorteilhaften Eigenschaften stellen die erfindungsgemässen Verbindungen eine Bereicherung der Pharmazie dar.

Je nach Art der verwendeten Ausgangsstoffe kann die Synthese der erfindungsgemässen Verbindungen beispielhaft durch folgende Formelschemata wiedergegeben werden:

A)

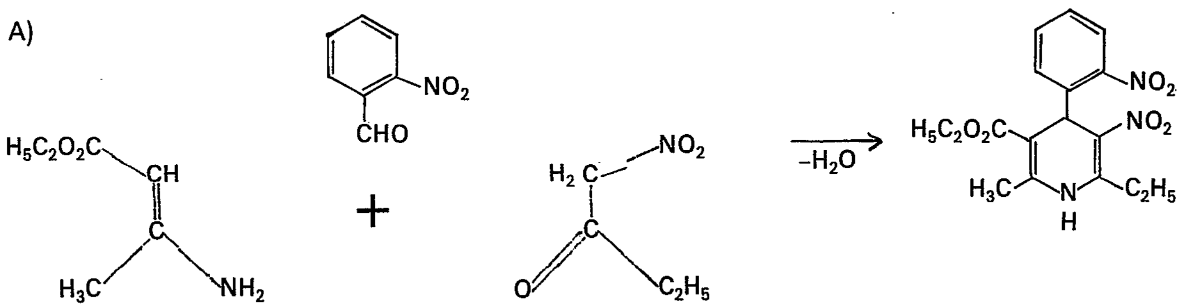


B)

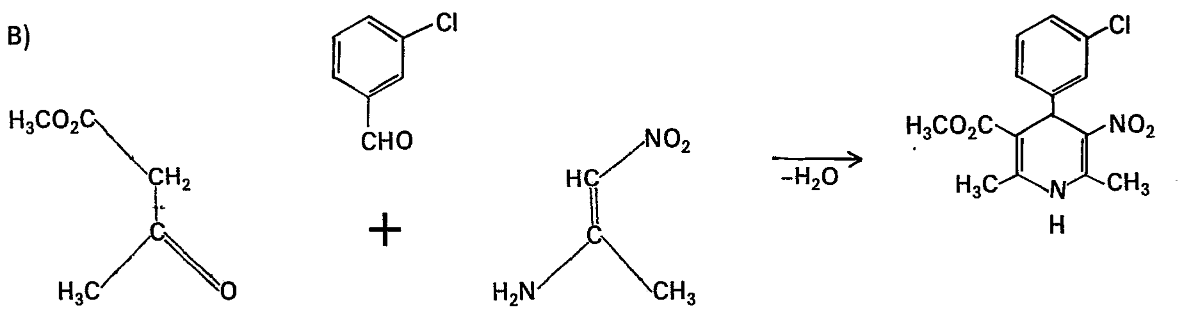


C)

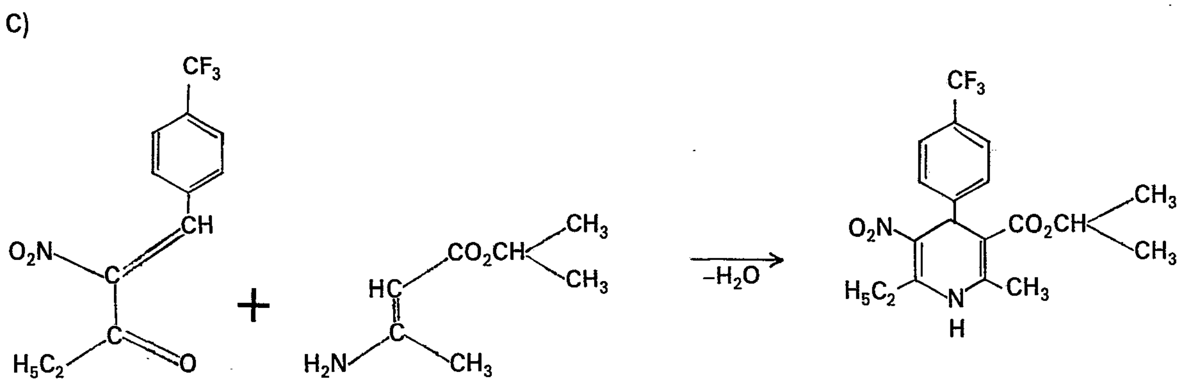

D)

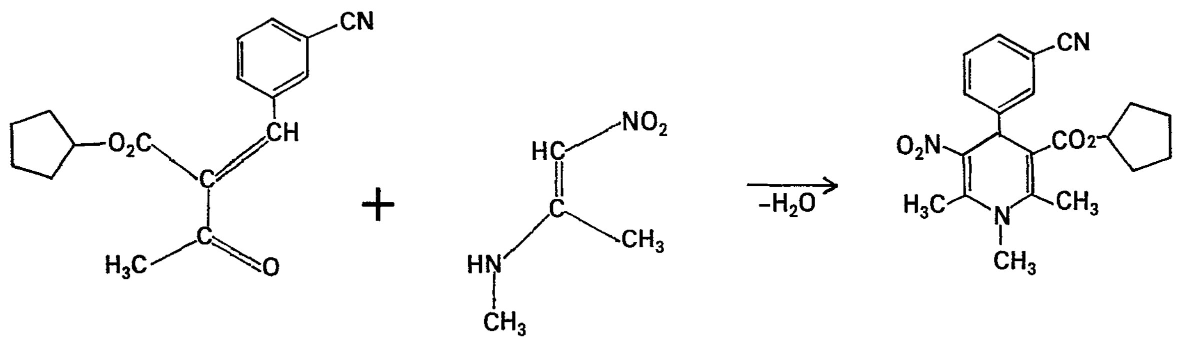


Verfahrensvariante A

Gemäss der unter A aufgegebenen Verfahrensweise wird ein Aldehyd der Formel

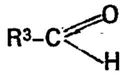 (II)

mit einem Enamin der Formel

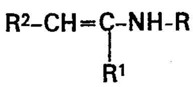 (III)

und einem Nitromethylketon der Formel

$R^4-CO-CH_2-NO_2$ (IV)

zur Reaktion gebracht.

Die als Ausgangsstoffe verwendeten Aldehyde der Formel II sind bekannt oder können nach bekannten Methoden hergestellt werden (vgl. z.B. E. Mosettig, Org. Reactions VIII, 218 ff [1954]).

Die als Ausgangssubstanzen verwendeten Enamine der Formel III sind bereits bekannt oder können nach bekannten Methoden hergestellt werden (vgl. A.C. Cope, J. Amer. chem. Soc. 67, 1017 [1945]).

Die als Ausgangsstoffe verwendeten Nitromethylketone der Formel IV sind teils bekannt oder können nach bekannten Methoden hergestellt werden (vgl. C.D. Hurd und M.E. Nilson, J. Org. Chem. 20, 927 ff [1955]; N. Levy und C.W. Scaife, J. Chem. Soc. London [1946], 1103).

Als Verdünnungsmittel kommen Wasser und inerte organische Lösungsmittel in Frage. Hierzu gehören vorzugsweise Alkohole wie Äthanol, Methanol, Isopropanol, Äther wie Dioxan, Diäthyläther, Tetrahydrofuran, Glykolmonomethyläther, Glykoldimethyläther oder Eisessig, Dimethylformamid, Dimethylsulfoxid, Acetonitril, Pyridin und Hexamethylphosphorsäuretriamid.

Die Reaktionstemperaturen können in einem grösseren Bereich variiert werden. Im allgemeinen arbeitet man zwischen 20°C und 150°C, vorzugsweise bei der Siedetemperatur des jeweiligen Lösungsmittels.

Die Umsetzung kann bei Normaldruck, aber auch bei erhöhtem Druck durchgeführt werden. Im allgemeinen arbeitet man unter Normaldruck.

Bei der Durchführung des erfindungsgemässen Verfahrens wird ein Mol Aldehyd der Formel II mit einem Mol Enaminverbindung der Formel III unter einem Mol Nitromethylketon der Formel IV in einem geeigneten Lösungsmittel zur Reaktion gebracht. Die Isolierung und Reinigung der erfindungsgemässen Substanzen erfolgt vorzugsweise derart, dass man das Lösungsmittel im Vakuum abdestilliert und das gegebenenfalls erst nach Chromatographie kristallin erhaltene Produkt aus einem geeigneten Lösungsmittel umkristallisiert.

Verfahrensvariante B

Gemäss der unter B angegebenen Verfahrensweise wird ein Aldehyd der Formel

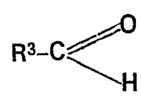 (II)

mit einer Carbonylverbindung der Formel

$R^1-CO-CH_2-R^2$ (V)

und einem Nitroenamin der Formel

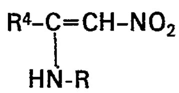 (VI)

zur Reaktion gebracht.

In den Formeln II, V und VI haben die Reste $R^1$, $R^2$, $R^3$ und $R^4$ die oben angegebene Bedeutung.

Die erfindungsgemäss verwendbaren Carbonylverbindungen der Formel V sind bereits bekannt oder können nach bekannten Methoden hergestellt werden (vgl. z.B. Houben-Weyl, Methoden der Organischen Chemie, VII/4, 230 ff [1968]; C.D. Hurd und M.E. Nilson, J. Org. Chem. 20, 927 ff [1955]).

Die als Ausgangsstoffe verwendeten N-subst. Nitroenamine der Formel VI sind teils bekannt oder können nach bekannten Methoden hergestellt werden (vgl. H. Böhme und K.-H. Weisel, Arch. Pharm. 310, 30-34 [1977]).

Vorzugsweise arbeitet man mit den gleichen Verdünnungsmitteln und unter den gleichen Reaktionsbedingungen wie unter Verfahrensvariante A.

Bei der Durchführung des erfindungsgemässen Vefahrens werden die an der Reaktion beteiligten Stoffe der Formel II, V und VI jeweils in molaren Mengen eingesetzt. Die erfindungsgemässen Verbindungen können leicht durch Umkristallisation aus einem geeigneten Lösungsmittel gereinigt werden.

Verfahrensvariante C

Gemäss Verfahren C wird ein Enamin der Formel

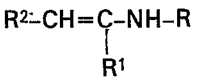 (III)

mit einer Nitroylidenverbindung der Formel

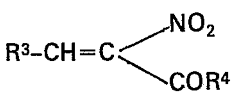 (VII)

zur Reaktion gebracht.

In den Formeln III und VII haben die Reste $R^1$, $R^2$, $R^3$ und $R^4$ die oben angegebene Bedeutung.

Die erfindungsgemäss verwendbaren Nitroylidenverbindungen der Formel VII sind teils bekannt oder können nach bekannten Methoden dargestellt werden (vgl. A. Dornow und W. Sassenberg, Liebigs Ann. Chem. 602, 14 f [1957]).

Vorzugsweise arbeitet man mit den gleichen Verdünnungsmitteln und unter den gleichen Reaktionsbedingungen wie unter Verfahrensvariante A.

Bei der Duchführung des erfindungsgemässen Verfahrens wird ein Mol der Nitroylidenverbindung der Formel VII mit einem Mol der Enaminverbindung der Formel III in einem geeigneten Lösungsmittel zur Reaktion gebracht. Die Isolierung und Reinigung der erfindungsgemässen Substanzen erfolgt vorzugsweise derart, dass man das Lösungsmittel im Vakuum abdestilliert und das gegebenenfalls erst nach Chromatographie kristallin erhaltene Produkt aus einem geeigneten Lösungsmittel umkristallisiert.

Verfahrensvariante D

Gemäss Verfahren D wird ein Nitroenamin der Formel

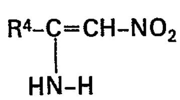 (VI)

mit einem Ylidenketon der Formel

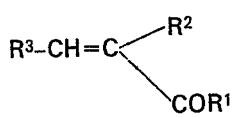 (VIII)

zur Reaktion gebracht.

In den Formel VI und VIII haben die Reste $R^1$, $R^2$, $R^3$ und $R^4$ die oben angegebene Bedeutung.

Die erfindungsgemäss verwendbaren Ylidenketone der Formel VIII sind teils bekannt oder können nach bekannten Methoden hergestellt werden (vgl. Org. Reactions XV, 204 ff [1967]).

Vorzugsweise arbeitet man mit den gleichen Verdünnungsmitteln und unter den gleichen Reaktionsbedingungen wie unter Verfahrensvariante A.

Bei der Durchführung des erfindungsgemässen Verfahrens werden die an der Reaktion beteiligten Stoffe der Formel VI und VIII jeweils etwa in molaren Mengen eingesetzt.

Die vorstehenden Herstellungsverfahren sind lediglich zur Verdeutlichung angegeben, und die Herstellung der Verbindungen der Formel I ist nicht auf diese Verfahren beschränkt, sondern jede Modifikation dieser Verfahren ist in gleicher Weise für die Herstellung der erfindungsgemässen Verbindungen anwendbar.

Je nach der Wahl der Ausgangssubstanzen können die erfindungsgemässen Verbindungen in steroisomeren Formen existieren, die sich entweder wie Bild und Spiegelbild (Enantiomere) oder die sich nicht wie Bild und Spiegelbild (Diastereomere) verhalten.

Sowohl die Antipoden als auch die Racemformen als auch die Diastereomerengemische sind Gegenstand der vorliegenden Erfindung. Die Racemformen lassen sich ebenso wie die Diastereomeren in bekannter Weise in die stereoisomer einheitlichen Bestandteile trennen (vgl. z. B. E.L. Eliel, Stereochemistry of Carbon Compounds, McGraw Hill, 1963).

Zusätzlich zu den unten aufgeführten Herstellungsbeispielen seien folgende erfindungsgemässen Wirkstoffe genannt:

1,4-Dihydro-2,6-dimethyl-3-nitro-4-phenyl-pyridin,

2-Äthyl-1,4-dihydro-6-methyl-3-nitro-4-phenylpyridin,

1,4-Dihydro-2,6-dimethyl-3-nitro-4-(2-nitrophenyl)-pyridin-5-carbonsäuremethylester,

4-(2-Chlorphenyl)-1,4-dihydro-2,6-dimethyl-3-nitro-pyridin-5-carbonsäureäthylester,

1,4-Dihydro-2,6-dimethyl-4-(3-nitrophenyl)-3-nitro-pyridin-5-carbonsäure-n-hexylester,

1,4-Dihydro-2,6-dimethyl-4-(2-nitrophenyl)-3-nitro-pyridin-5-carbonsäure-t-butylester,

4-(3,4-Dichlorphenyl)-1,4-dihydro-2,6-dimethyl-3-nitropyridin-5-carbonsäure-i-propylester,

4-(2-Chlor-5-nitrophenyl)-1,4-dihydro-2,6-dimethyl-3-nitropyridin-5-carbonsäure-n-propylester,

4-(2-Cyanophenyl)-1,4-dihydro-2,6-dimethyl-3-nitro-pyridin-5-carbonsäureäthylester,

4-(3-Cyanophenyl)-1,4-dihydro-2,6-dimethyl-3-nitro-pyridin-5-carbonsäure-(β-methoxyäthyl)-ester,

1,4-Dihydro-2,6-dimethyl-3,5-dinitro-4-(2-nitrophenyl)-pyridin,

2-Äthyl-1,4-dihydro-3,5-dinitro-6-methyl-4-(2-trifluormethylphenyl)-pyridin.

Die neuen Verbindungen sind als Arzneimittel verwendbare Substanzen. Sie haben ein breites und vielseitiges pharmakologisches Wirkungsspektrum.

Im einzelnen konnten im Tierexperiment folgende Hauptwirkung nachgewiesen werden:

1) Die Verbindungen bewirken bei parenteraler, oraler und perlingualer Zugabe eine deutliche und langanhaltende Erweiterung der Coronargefässe. Diese Wirkung auf die Coronargefässe wird durch einen gleichzeitigen nitritähnlichen herzentlastenden Effekt verstärkt.

Sie beeinflussen bzw. verändern den Herzstoffwechsel im Sinne einer Energieersparnis.

2) Die Erregbarkeit des Reizbildungs- und Erregungsleitungssystems innerhalb des Herzens wird herabgesetzt, so dass eine in therapeutischen Dosen nachweisbare Antiflimmerwirkung resultiert.

3) Der Tonus der glatten Muskulatur der Gefässe wird unter der Wirkung der Verbindungen stark vermindert. Diese gefässspasmolytische Wirkung kann im gesamten Gefässsystem stattfinden, oder sich mehr oder weniger isoliert in umschriebenen Gefässgebieten (z.B. dem Zentralnervensystem manifestieren.

4) Die Verbindungen senken den Blutdruck von normotonen und hypertonen Tieren und können somit als antihypertensive Mittel verwendet werden.

5) Die Verbindungen haben stark muskulärspasmolytische Wirkungen, die an der glatten Muskulatur des Magens, Darmtraktes, des Urogenitaltraktes und des Respirationssystems deutlich werden.

Die neuen Wirkstoffe können in an sich bekannter Weise in die üblichen Formulierungen übergeführt werden, wie Tabletten, Kapseln, Dragees, Pillen, Granulate, Aerosole, Sirupe, Emulsionen, Suspensionen und Lösungen, unter Verwendung inerter, nichttoxischer pharmazeutisch geeigneter Trägerstoffe oder Lösungsmittel. Hierbei soll die therapeutisch wirksame Verbindung jeweils in einer Konzentration von etwa 0,5 bis 90 Gewichtsprozent der Gesamtmischung vorhanden sein, d.h. in Mengen, die ausreichend sind, um den angegebenen Dosierungsspielraum zu erreichen.

Die Formulierungen werden beispielsweise hergestellt durch Verstrecken der Wirkstoffe mit Lösungsmitteln und/oder Trägerstoffen, gegebenenfalls unter Verwendung von Emulgiermitteln und/oder Dispergiermitteln, wobei z.B. im Fall der Benutzung von Wasser als Verdünnungsmittel gegebenenfalls organische Lösungsmittel als Hilfslösungsmittel verwendet werden können.

Als Hilfsstoffe seien beispielhaft aufgeführt: Wasser, nichttoxische organische Lösungsmittel, wie Paraffine (z.B. Erdölfraktionen), pflanzliche Öle (z.B. Erdnuss-/Sesamöl), Alkohole (z.B. Äthylalkohol, Glycerin), Glykole (z.B. Propylenglykol), feste Trägerstoffe, wie natürliche Gesteinsmehle (z.B. Kaoline, Tonerden, Talkum, Kreide), synthetische Gesteinsmehle (z.B. hochdisperse Kieselsäure, Silikate), Zucker (z.B. Roh-, Milche- und Traubenzucker), Emulgiermittel, wie nichtionogene anionische Emulgatoren (z.B. Polyoxyäthylen-Fettsäure-Ester, Polyoxyäthylen-Fettalkohol-Äther, Alkylsulfonate und Arylsulfonate), Dispergiermittel (z.B. Lignin, Sulfitablaugen, Methylcellulose, Stärke und Polyvinylpyrrolidon) und Gleitmittel (z.B. Magnesiumstearat, Talkum, Stearinsäure und Natriumlaurylsulfat).

Die Applikation erfolgt in üblicher Weise, vorzugsweise oral oder parenteral, insbesondere perlingual oder intravenös. Im Falle der oralen Anwendung können Tabletten ausser den genannten Trägerstoffen auch Zusätze, wie Natriumcitrat, Calciumcarbonat und Dicalciumphosphat zusammen mit verschiedenen Zuschlagstoffen, wie Stärke, vorzugsweise Kartoffelstärke und Gelatine enthalten. Weiterhin können Gleitmittel, wie Magnesiumstearat, Natriumlaurylsulfat und Talkum zum Tablettieren mitverwendet werden. Im Falle wässriger Suspensionen und/oder Elixieren, die für orale Anwendungen gedacht sind, können die Wirkstoffe ausser mit den obengenannten Hilfsstoffen mit verschiedenen Geschmacksaufbesserern oder Farbstoffen versetzt werden.

Für den Fall der parenteralen Anwendung können Lösungen der Wirkstoffe unter Verwendung geeigneter flüssiger Trägermaterialien eingesetzt werden.

Im allgemeinen hat es sich als vorteilhaft erwiesen, bei intravenöser Applikation Mengen von etwa 0,01 bis 10 mg/kg vorzugsweise etwa 0,05 bis 5 mg/kg, Körpergewicht pro Tag zur Erzielung wirksamer Ergebnisse zu verabreichen, und bei oraler Applikation beträgt die Dosierung etwa 0,05 bis 20 mg/kg, vorzugsweise 0,5 bis 5 mg/kg, Körpergewicht pro Tag.

Trotzdem kann es erforderlich sein, von den genannten Mengen abzuweichen, und zwar in Abhängigkeit vom Körpergewicht des Versuchstieres bzw. der Art der Applikationswege, aber auch aufgrund der Tierart und deren individuellem Verhalten gegenüber dem Medikament bzw. der Art von dessen Formulierung und dem Zeitpunkt bzw. Intervall, zu welchem die Verabreichung erfolgt. So kann es in einigen Fällen ausreichend sein, mit weniger als der vorgenannten Mindestmenge auszukommen, während in anderen Fällen die genannte obere Grenze überschritten werden muss. Im Falle der Applikation grösserer Mengen kann es empfehlenswert sein, diese in mehrere Einzelgaben über den Tag zu verteilen. Für die Applikation in der Humanmedizin ist der gleiche Dosierungsspielraum vorgesehen. Sinngemäss gelten hierbei auch die obigen Ausführungen.

## HERSTELLUNGSBEISPIELE

Beispiel 1

1.4-Dihydro-2,6-dimethyl-3-nitro-4-(3-nitrophenyl)-pyridin-5-carbonsäuremethylester

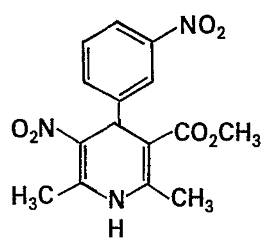

Verfahrensvariante A

11,5 g (0,1 Mol) 3-Nitrobenzaldehyd werden zusammen mit 11,5 g (0,1 Mol) β-Aminocrotonsäuremethylester und 10,3 g (0,1 Mol) Nitroaceton in 150 ml Äthanol 12 Std. unter Rückfluss erhitzt. Nach dem Erkalten der Reaktionsmischung wurde das Lösungsmittel im Vakuum abdestilliert, der ölige Rückstand in wenig Chloroform aufgenommen und auf einer Kieselgelsäule (0,2–0,5 mm Korngrösse) mit Chloroform unter Zusatz von 3% Methanol chromatographiert.

Die das Reaktionsprodukt enthaltenden Fraktionen wurden eingeengt, der Rückstand in wenig Isopropanol aufgenommen. Das Nitrodihydropyridin kristallisierte in gelben Kristallen vom Smp. 204–206 °C.
Ausbeute: 10,3 g (31% d. Th.).

Verfahrensvariante D

24,9 g (0,1 Mol) 3-Nitrobenzylidenacetessigsäuremethylester wurden zusammen mit 10,2 g (0,1 Mol) 2-Amino-1-nitro-1-propen in 100 ml Äthanol für 8 Std. unter Rückfluss erhitzt. Nach dem Abkühlen der Reaktionsmischung wurde das Lösungsmittel im Vakuum abdestilliert und der Rückstand aus Isopropanol kristallisiert; gelbe Kristalle, Smp.: 204–206 °C.
Ausbeute: 17,2 g (52% d. Th.).

Beispiel 2

1.4-Dihydro-2,6-dimethyl-3-nitro-4-(3-nitrophenyl)-pyridin-5-carbonsäureäthylester

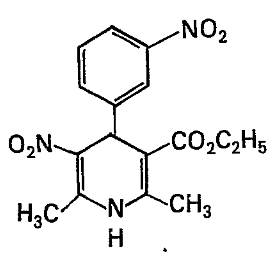

Verfahrensvariante B

15,1 g (0,1 Mol) 3-Nitrobenzaldehyd werden zusammen mit 13,0 g (0,1 Mol) Acetessigsäureäthylester und 10,2 g (0,1 Mol) 2-Amino-1-nitro-1-propen in 150 ml Äthanol für 12 Std. unter Rückfluss erhitzt. Das Lösungsmittel wurde nach dem Erkalten der Reaktionsmischung im Vakuum abdestilliert, der Rückstand in wenig Chloroform aufgenommen und auf eine Kieselgelsäule (0,2–0,5 mm Korngrösse) aufgetragen. Es wurde mit Chloroform unter Zusatz von 3% Methanol eluiert.

Die das Reaktionsprodukt enthaltenden Fraktionen wurden eingeengt, das Produkt dann aus Isopropanol umkristallisiert. Man erhielt gelbe Prismen vom Smp. 215 °C.
Ausbeute: 8,3 g (24% d.Th.).

Beispiel 3

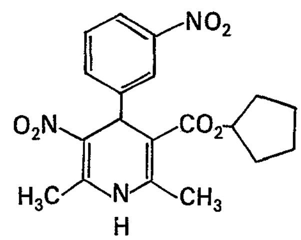

Verfahrensvariante B

Analog Beispiel 2 wurde durch Umsetzung von 0,1 Mol 3-Nitrobenzaldehyd, 0,1 Mol 2-Amino-1-nitro-1-propen und 0,1 Mol Acetessigsäurecyclopentylester in Äthanol 1.4-Dihydro-2,6-dimethyl-3-nitro-4-(3-nitrophenyl)-pyridin-5-carbonsäure-cyclopentylester erhalten.
Smp.: 174 °C (Isopropanol)
Ausbeute: 37% d.Th.

Verfahrensvariante C

23,6 g (0,1 Mol) 2-Nitro-1-(3-nitrophenyl)-buten-1-on-3 werden zusammen mit 16,9 g (0,1 Mol) β-Amino-crotonsäure-cyclopentylester in 150 ml Äthanol für 8 Std. unter Rückfluss erhitzt. Das Lösungsmittel wurde anschliessend im Vakuum abdestilliert, der Rückstand kristallisierte aus wenig Isopropanol in gelben Kristallen vom Smp. 174 °C.
Ausbeute: 18,5 g (48% d.Th.).

Beispiel 4

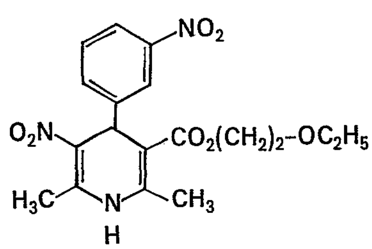

Analog Beispiel 2 B wurde durch Umsetzung von 3-Nitrobenzaldehyd mit 0,1 Mol Acetessigsäure-(β-äthoxyäthyl)-ester und 2-Amino-1-nitro-1-propen in Äthanol 1.4-Dihydro-2,6-dimethyl-3-nitro-4-(3-nitrophenyl)-pyridin-5-carbonsäure-(β-äthoxyäthyl)-ester vom Smp. 140 °C (Isopropanol) erhalten.
Ausbeute: 36% d.Th.

Analog Beispiel 3 C wurde durch Umsetzung von 2-Nitro-1-(3-nitrophenyl)-buten-1-on-3 mit β-Aminocrotonsäure-(β-äthoxyäthyl)-ester in Äthanol 1.4-Dihydro-2,6-dimethyl-3-nitro-4-(3-nitrophenyl)-pyridin-5-carbonsäure-(β-äthoxyäthyl)-ester vom Smp. 140 °C (Isopropanol) erhalten.
Ausbeute: 48% d.Th.

Beispiel 5

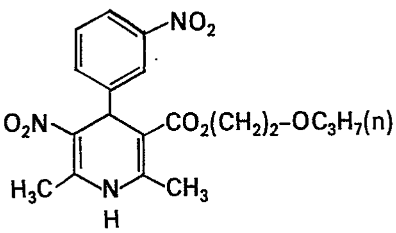

Analog Beispiel 2 B wurde durch Umsetzung von 3-Nitrobenzaldehyd mit 0,1 Mol Acetessigsäure-(β-n-propoxyäthyl)-ester und 2-Amino-1-nitro-1-propen in Äthanol 1.4-Dihydro-2,6-dimethyl-3-nitro-4-(3-nitrophenyl)-pyridin-5-carbonsäure-(β-n-propoxyäthyl)-ester vom Smp. 161 °C (Isopropanol) erhalten.
Ausbeute: 41% d.Th.

Beispiel 6

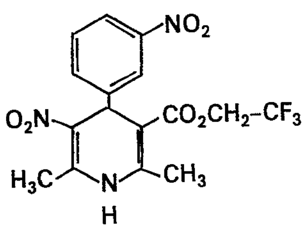

Analog Beispiel 1 D wurde durch Umsetzung von 3-Nitrobenzyliden-acetessigsäure-(β-trifluoräthyl)-ester mit 2-Amino-1-nitro-1-propen in Äthanol 1.4-Dihydro-2,6-dimethyl-3-nitro-4-(3-nitrophenyl)-pyridin-5-carbonsäure-(β-trifluoräthyl)-ester vom Smp. 196 °C (Äthanol) erhalten.
Ausbeute: 28% d.Th.

Beispiel 7

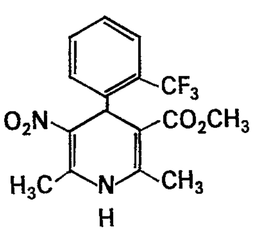

Analog Beispiel 1 A wurde durch Umsetzung von 2-Trifluormethyl-benzaldehyd mit β-Aminocrotonsäuremethylester und Nitroaceton in Äthanol 1.4-Dihydro-2,6-dimethyl-3-nitro-4-(2-trifluormethylphenyl)-pyridin-5-carbonsäuremethylester vom Smp. 176 °C (Äthanol) erhalten.
Ausbeute: 36% d.Th.

Analog Beispiel 1 D wurde durch Umsetzung von 2-Trifluormethyl-benzylidenacetessigsäuremethylester mit 2-Amino-1-nitro-1-propen in Äthanol 1.4-Dihydro-2,6-dimethyl-3-nitro-4-(2-trifluor-methylphenyl)-pyridin-5-carbonsäuremethylester vom Smp. 176 °C (Äthanol) erhalten.
Ausbeute: 47% d.Th.

Beispiel 8

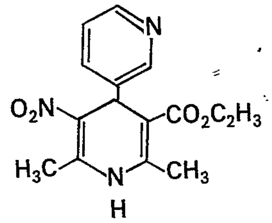

Analog Beispiel 1 A wurde durch Umsetzung von Pyridin-3-aldehyd mit β-Aminocrotonsäureäthylester und Nitroaceton in Äthanol 1.4-Dihydro-2,6-dimethyl-3-nitro-4-(pyridyl-3-)-pyridin-5-carbonsäureäthylester vom Smp. 264 °C (Isopropanol) erhalten.
Ausbeute: 34% d.Th.

Beispiel 9

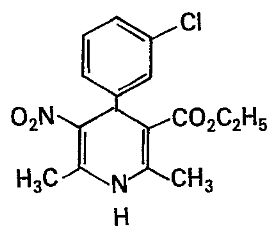

Analog Beispiel 3 C wurde durch Umsetzung von 1-(3-Chlorphenyl)-2-nitro-buten-1-on-3 mit β-Aminocrotonsäureäthylester in Äthanol 4-(3-Chlorphenyl)-1.4-dihydro-2,6-dimethyl-3-nitro-pyridin-5-carbonsäureäthylester vom Smp. 168 °C (Isopropanol) erhalten.
Ausbeute: 52% d.Th.

Analog Beispiel 1 D wurde durch Umsetzung von 3-Chlorbenzylidenacetessigsäureäthylester mit 2-Amino-1-nitro-1-propen in Äthanol 4-(3-Chlorphenyl)-1.4-dihydro-2,6-dimethyl-3-nitro-pyridin-5-carbonsäureäthylester vom Smp. 168 °C (Isopropanol) erhalten.
Ausbeute: 48% d.Th.

Beispiel 10

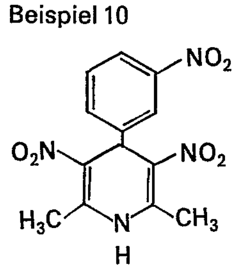

Analog Beispiel 1 A wurde durch Umsetzung von 3-Nitrobenzaldehyd mit Nitroaceton und 2-Amino-1-nitro-1-propen in Äthanol 1.4-Dihydro-2,6-dimethyl-3,5-dinitro-4-(3-nitrophenyl)-pyridin vom Smp. 237–240 °C unter Zersetzung (Isopropanol) erhalten.
Ausbeute: 38% d.Th.

Analog Beispiel 1 D wurde durch Umsetzung von 1-Nitro-1-(3-nitrophenyl)-buten-1-on-3 mit

2-Amino-1-nitro-1-propen in Äthanol 1.4-Dihydro-2,6-dimethyl-3,5-dinitro-4-(3-nitrophenyl)-pyridin vom Smp. 237–240 °C unter Zersetzung (Isopropanol) erhalten.
Ausbeute: 47% d.Th.

**Patentansprüche**

1. Nitrosubstituierte 1,4-Dihydropyridine der allgemeinen Formel

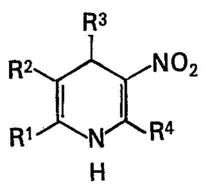

(I)

in welcher
$R^1$ und $R^4$ für einen Alkylrest mit 1 oder 2 Kohlenstoffatomen,
$R^2$ für eine Nitrogruppe oder für die Gruppe $COOR^6$ steht, wobei $R^6$ ein geradkettiges, verzweigtes oder cyclisches Alkyl, Alkenyl oder Alkinyl mit bis zu 6 Kohlenstoffatomen bedeutet, und die Alkylgruppe gegebenenfalls durch ein Sauerstoffatom unterbrochen ist oder wobei die Alkylgruppe gegebenenfalls durch Halogen substituiert ist, und
$R^3$ für einen Phenylrest, der gegebenenfalls durch 1 oder 2 gleiche oder verschiedene Halogenatome, Nitro-, Cyano- oder Trifluormethylgruppen substituiert ist, oder für einen Pyridylrest stehen.

2. Verfahren zur Herstellung von nitrosubstituierten 1,4-Dihydropyridinen der allgemeinen Formel I gemäss Anspruch 1, dadurch gekennzeichnet, dass man
A) Aldehyde der Formel

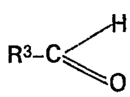

(II)

in welcher $R^3$ die oben angegebene Bedeutung hat
mit
Enaminen der Formel

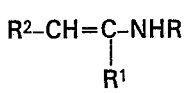

(III)

in welcher R, $R^1$ und $R^2$ die oben angegebene Bedeutung haben
und
Nitromethylketonen der Formel

$R^4-CO-CH_2-NO_2$ (IV)

in welcher $R^4$ die oben angegebene Bedeutung hat in Wasser oder in inerten organischen Lösungsmitteln umsetzt,
oder
B) Aldehyde der Formel

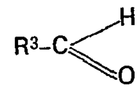

(II)

in welcher $R^3$ die oben angegebene Bedeutung hat
mit
Carbonylverbindungen der Formel

$R^1-CO-CH_2-R^2$ (V)

in welcher $R^1$ und $R^2$ die oben angegebene Bedeutung haben
und
Nitroenaminen der Formel

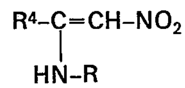

(VI)

in welcher R und $R^4$ die oben angegebene Bedeutung besitzen
in
Wasser oder inerten organischen Lösungsmitteln umsetzt
oder
C) Enamine der Formel

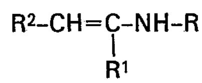

(III)

in welcher R, $R^1$ und $R^2$ die oben angegebene Bedeutung haben
mit
Nitroylidenverbindungen der Formel

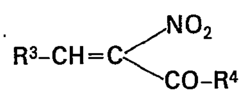

(VII)

in welcher $R^3$ und $R^4$ die oben angegebene Bedeutung haben
in Wasser oder inerten organischen Lösungsmitteln umsetzt
oder
D) Nitroenamine der Formel

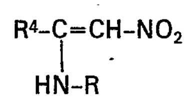

(VI)

in welcher R und $R^4$ die oben angegebene Bedeutung haben
mit
Ylidenketonen der Formel

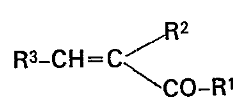

(VIII)

in welcher $R^1$, $R^2$ und $R^3$ die oben angegebene Bedeutung haben
in Wasser oder inerten organischen Lösungsmitteln umsetzt.

3. Arzneimittel enthaltend nitrosubstituierte 1,4-Dihydropyridine gemäss Anspruch 1.

4. Verfahren zur Herstellung von Arzneimitteln, dadurch gekennzeichnet, dass man nitrosubstituierte 1,4-Dihydropyridine gemäss Anspruch 1, gegebenenfalls unter Zusatz von inerten pharmazeutisch unbedenklichen Hilfs- und Trägerstoffen in eine geeignete Applikationsform überführt.

**Patent Claims**

1. Nitro-substituted 1,4-dihydropyridines of the general formula

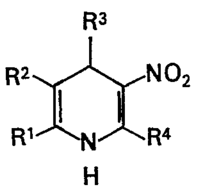

(I)

in which

$R^1$ and $R^4$ represent an alkyl radical with 1 or 2 carbon atoms,

$R^2$ represents a nitro group or the group $COOR^6$, in which $R^6$ denotes a straight-chain, branched or cyclic alkyl, alkenyl or alkinyl with up to 6 carbon atoms, and the alkyl group is optionally interrupted by an oxygen atom or in which the alkyl group is optionally substituted by halogen and

$R^3$ represents a phenyl radical which is optionally substituted by 1 or 2 identical or different halogen atoms, nitro groups, cyano groups or trifluoromethyl groups, or it represents a pyridyl radical.

2. Process for the preparation of nitro-substituted 1,4-dihydropyridines of the general formula I according to claim 1, characterised in that

A) aldehydes of the formula

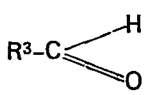

(II)

in which $R^3$ has the above-mentioned meaning are reacted with enamines of the formula

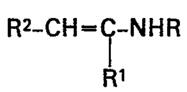

(III)

in which R, $R^1$ and $R^2$ have the above-mentioned meaning

and

nitromethyl ketones of the formula

$$R^4\text{-}CO\text{-}CH_2\text{-}NO_2 \qquad (IV)$$

in which $R^4$ has the above-mentioned meaning, in water or in inert organic solvents,

or

B) aldehydes of the formula

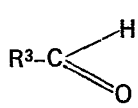

(II)

in which $R^3$ has the above-mentioned meaning are reacted with carbonyl compounds of the formula

$$R^1\text{-}CO\text{-}CH_2\text{-}R^2 \qquad (V)$$

in which $R^1$ and $R^2$ have the above-mentioned meaning

and

nitroenamines of the formula

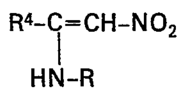

(VI)

in which R and $R^4$ have the above-mentioned meaning in water or inert organic solvents or

C) enamines of the formula

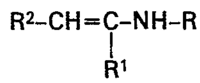

(III)

in which R, $R^1$ and $R^2$ have the above-mentioned meaning are reacted with nitroylidene compounds of the formula

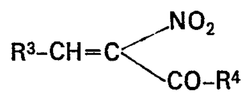

(VII)

in which $R^3$ and $R^4$ have the above-mentioned meaning in water or inert organic solvents

D) nitroenamines of the formula

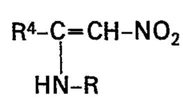

(VI)

in which R and $R^4$ have the above-mentioned meaning are reacted with ylidene ketones of the formula

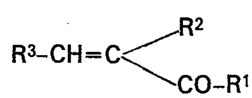

(VIII)

in which $R^1$, $R^2$ and $R^3$ have the above-mentioned meaning in water or inert organic solvents.

3. Medicaments containing nitrosubstituted 1,4-dihydropyridines according to claim 1.

4. Process for the production of medicaments, characterised in that nitrosubstituted 1,4-dihydropyridines according to claim 1 are converted into a suitable application form, optionally by adding inert pharmaceutically acceptable auxiliaries and carriers.

**Revendications**

1. 1,4-Dihydropyridines nitrosubstituées, caractérisées en ce qu'elles répondent à la formule générale

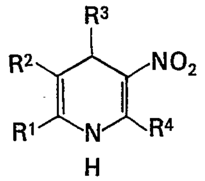

(I)

dans laquelle
$R^1$ et $R^4$ représentent un reste alkyle en $C_1$ ou $C_2$,
$R^2$ représente un groupe nitro ou le groupe $COOR^6$, où $R^6$ représente un groupe alkyle, alcényle ou alcynyle à chaîne droite ou ramifiée jusqu'en $C_6$ et le groupe alkyle est éventuellement interrompu par un atome d'oxygène ou le groupe alkyle est éventuellement substitué par un halogène, et
$R^3$ représente un reste phényle qui est éventuellement substitué par un ou deux atomes d'halogènes identiques ou différents ou groupes nitro, cyano ou trifluorométhyle, ou un reste pyridyle.

2. Procédé pour la préparation des 1,4-dihydropyridines nitrosubstituées de formule générale (I) selon la revendication 1, caractérisé en ce que:
A) on fait réagir des aldéhydes de formule

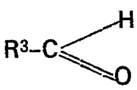

(II)

dans laquelle $R^3$ a la signification indiquée ci-dessus, avec des èneamines de formule

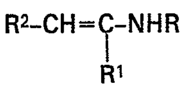

(III)

dans laquelle $R^1$ et $R^2$ ont la signification indiquée ci-dessus et des nitrométhylcétones de formule

$R^4$-CO-$CH_2$-$NO_2$ (IV)

dans laquelle $R^4$ a la signification indiquée ci-dessus, dans l'eau ou dans des solvants organiques inertes, ou bien
B) on fait réagir des aldéhydes de formule

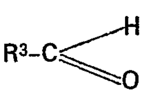

(II)

dans laquelle $R^3$ a la signification indiquée ci-dessus, avec des composés carbonylés de formule

$R^1$-CO-$CH_2$-$R^2$ (V)

dans laquelle $R^1$ et $R^2$ ont la signification indiquée ci-dessus et des nitroèneamines de formule

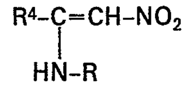

(VI)

dans laquelle R et $R^4$ ont la signification indiquée ci-dessus dans l'eau ou dans des solvants organiques inertes, ou bien
C) on fait réagir des èneamines de formule

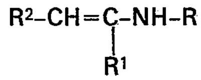

(III)

dans laquelle R, $R^1$ et $R^2$ ont la signification indiquée ci-dessus, avec des composés nitroylidéniques de formule

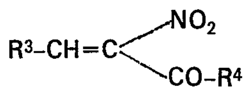

(VII)

dans laquelle $R^3$ et $R^4$ ont la signification indiquée ci-dessus, dans l'eau ou dans des solvants organiques inertes, ou bien
D) on fait réagir des nitroèneamines de formule

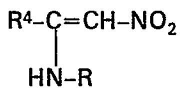

(VI)

dans laquelle R et $R^4$ ont la signification indiquée ci-dessus, avec des ylidènecétones de formule

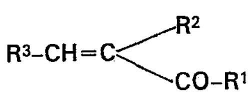

(VIII)

dans laquelle $R^1$, $R^2$ et $R^3$ ont la signification indiquée ci-dessus, dans l'eau ou dans des solvants organiques inertes.

3. Médicaments contenant des 1,4-dihydropyridines nitrosubstituées selon la revendication 1.

4. Procédé pour la préparation de médicaments, caractérisée en ce que l'on transforme des 1,4-dihydropyridines nitrosubstituées selon la revendication 1 en une forme d'application appropriée, éventuellement avec addition d'agents auxiliaires et de supports inertes inoffensifs en pharmacie.